# FASCICULE DE BREVET EUROPEEN

(11) **EP 1 367 944 B1**
(45) Date de publication et mention de la délivrance du brevet: **13.10.2004**
(21) Numéro de dépôt: 02708443.3
(22) Date de dépôt: 06.03.2002
(51) Int. Cl.: A61B 10/00

(54) **PATCH DESTINE NOTAMMENT A DEPISTER L'ETAT DE SENSIBILISATION D'UN SUJET A UN ALLERGENE**
PFLASTER ZUR BESTIMMUNG DES ZUSTANDS DER SENSIBILISIERUNG EINES SUBJEKTS FÜR EIN ALLERGEN
PATCH FOR SCREENING THE SENSITIVITY STATE OF A SUBJECT WITH RESPECT TO AN ALLERGEN

(30) Priorité: 13.03.2001 FR 0103382
(43) Date de publication de la demande: 10.12.2003
(73) Titulaire: DBV Technologies, 92100 Boulogne (FR)
(72) Inventeur: DUPONT, Christophe, F-92140 Clamart (FR); DUPONT, Bertrand, F-13100 Aix En Provence (FR); VANNEROM, Pierre-Yves, F-75017 Paris (FR); BENHAMOU, Stéphane, 97133 Saint Barthelemy (FR); BENHAMOU, Pierre-Henri, F-75015 Paris (FR)
(74) Mandataire: Vuillermoz, Bruno
(86) Numéro de dépôt international: PCT/FR2002/000804
(87) Numéro de publication internationale: WO 2002/071950

(56) Documents cités:
- WO-A-96/32142
- US-A- 3 837 340
- US-A- 3 894 531
- US-A- 4 450 844
- US-A- 4 788 971

## Description

L'invention concerne une nouvelle forme de patch apte à délivrer une substance biologiquement active au niveau de l'épiderme. Elle a en particulier pour objet, un patch destiné notamment à dépister l'état de sensibilisation d'un sujet à un allergène. Elle se rapporte également à l'utilisation dudit patch notamment pour le dépistage de l'état de sensibilisation d'un sujet à un allergène.

Dans la suite de la description, par « substance biologiquement active », on désigne une substance à visée diagnostique, thérapeutique, cosmétique ou préventive (par exemple vaccin) se présentant sous forme de particules individualisées ou agglomérées.

L'invention sera plus particulièrement décrite en relation avec les allergènes, considérés, au sens de la définition qui précède comme des substance à visée diagnostique, c'est à dire des substances permettant de dépister l'état de sensibilisation d'un sujet à un allergène donné.

Dans la suite de la description, par l'expression « allergène de contact », on désigne tout allergène susceptible d'entraîner une réaction au contact direct de la peau sans aucune réaction à distance, lorsque ledit allergène est mis au contact de l'organisme d'un sujet. On trouve ce type d'allergène dans un certain nombre de produits naturels ou synthétiques, qui lorsqu'ils sont mis au contact de la peau d'un sujet, entraînent une allergie dite « de contact » provoquant une réaction cutanée locale se caractérisant par différents phénomènes tels que éruption, démangeaisons, apparition de vésicules et eczéma. De tels allergènes sont parfaitement connus de l'homme du métier et sont précisément listés dans la littérature, en particulier dans le document US-A-4836217. On connaît de nombreuses allergies de contact et notamment les allergies au métaux, tels que par exemple le nickel contenu dans les bracelets de montre, le chrome contenu dans le ciment, mais également les allergies aux parfums, à la lanoline contenus dans les produits cosmétiques, les allergies au substances actives, telles que la néomycine, la flavine contenus dans certains médicaments etc..

La présente invention concerne non seulement les allergies de contact, mais également et surtout toutes les manifestations allergiques susceptibles de se manifester non pas exclusivement par une réaction cutanée au contact de l'allergène, mais également par un certain nombre de symptômes siégeant à distance du lieu de contact avec l'allergène par exemple choc anaphylactique, diarrhée, sinusite, asthme, eczéma généralisé, urticaire, etc... Il en est ainsi des allergies aux acariens, pollens, poils d'animaux, aliments divers et diverses substances d'origine végétale ou animale. De nombreux allergènes sont en cause, ainsi par exemple, les acariens, les pollens, les poils ou plumes d'animaux etc. que l'on qualifie parfois d'allergènes «respiratoires » sont à l'origine de manifestations respiratoires du type rhinites ou asthmes. De même, l'arachide, l'oeuf, le lait, le blé, que l'on qualifie parfois d'allergènes « alimentaires » sont à l'origine de pathologies digestives, telles que la diarrhée chronique de l'enfant ou de pathologies anaphylactiques de type choc, en réponse par exemple à l'ingestion d'arachide. L'allergie au latex est également parfaitement connue et entraîne des symptômes du type anaphylactique faisant courir au patient des risques per-opératoires potentiellement graves. La majorité de ces allergènes-est précisément listée dans le document EP-A-107832.

Les moyens de diagnostic de l'allergie sont fondés sur un faisceau de tests au premier rang desquels se situent les tests cutanés. L'apparition d'une réactivité de la peau au contact de l'allergène se traduit par une réaction d'inflammation locale cutanée, soit modérée sous forme d'érythème (premier élément clinique de la réaction inflammatoire), soit sous forme d'une papule signant également la présence d'un oedème local (autre composant de la réaction inflammatoire). La réactivité cutanée d'un allergène autre qu'un allergène de-contact s'explique par la circulation permanente des éléments immunologiques dans le sang, permettant aux lymphocytes sensibilisés par les allergènes entrés dans l'organisme par les voies respiratoires ou digestives, de siéger également au niveau de la peau.

Plusieurs tests cutanés sont aujourd'hui proposés pour détecter l'état de sensibilisation d'un individu vis à vis des allergènes de contact aussi bien que des allergènes respiratoires et alimentaires.

Parmi ceux-ci, on connaît notamment le test désigné sous la dénomination « Prick Test », lequel concerne tous les allergènes susceptibles de déclencher une réaction cutanée immédiate aux allergènes alimentaires ou respiratoires. Au cours de ce test, une solution contenant l'allergène est déposée sur la peau, puis ledit allergène est mis en contact avec les éléments immunologiques au moyen d'un stylet perforant la partie superficielle du derme à travers la solution. La lecture du Prick Test est effectuée après une demi-heure de contact du derme avec l'allergène. En d'autres termes et comme déjà dit, ce test permet de détecter une réaction immédiate, en générale IgE dépendante, c'est-à-dire mettant en oeuvre une réactivité immunoglobuline de type E. La lecture se fait par-comparaison avec un témoin positif, tel que l'histamine et un témoin négatif, sérum salé ou solvant utilisé pour la dilution des allergènes. L'inconvénient du Prick Test réside d'une part, dans son caractère douloureux, du fait de la perforation du derme avec le stylet ou l'aiguille , et d'autre part et surtout, dans l'exploration exclusive d'une réactivité immédiate.

Or, il apparaît qu'un certain nombre de réactions allergiques surviennent de façon retardée ou semi-retardée dans un délai de plusieurs heures à plusieurs jours. On a par ailleurs constaté que le simple contact de la peau avec un allergène provoquait l'apparition de réactions systémiques. On en déduit donc que l'allergène diffuse à travers la peau de sorte qu'il peut déclencher des réactions immédiates tout comme des réactions retardées.

A partir de ce constat, on a proposé de déposer les allergènes sur des supports destinés à être maintenus au contact de la peau de façon durable, de manière à permettre le passage de l'allergène à travers la peau et ainsi de déclencher une réaction cutanée. Deux principaux types de test ont été développés et sont connus sous le terme générique de « patch test ».

Un premier patch est connu sous la dénomination FINN CHAMBERS®. Il comprend un support adhésif sur lequel sont collées de petites coupelles métalliques d'un centimètre de diamètre environ. Ces coupelles de 2 à 3 millimètres de profondeur reçoivent des allergènes dilués, déposés sur un support de cellulose non solidaire de la coupelle, le mélange étant préparé extemporanément à partir du produit natif ou d'allergènes en suspension. Le support cellulosique est déposé au fond de la coupelle et la coupelle est ensuite fixée sur la peau du patient. La lecture du test est effectuée au bout de 48 heures après ablation du matériel, nettoyage de la peau et petit délai d'attente, une demi-heure environ pour permettre la disparition des phénomènes locaux spécifiques, liés à la pression du bord de la cupule sur la peau ou à la présence de l'adhésif. La réaction positive associe érythème, oedème et éruption maculaire au point de contact. Elle est comparée à celle provoquée par un témoin négatif (support de cellulose simplement humidifié à l'eau). L'interprétation est en général facile, mais la réaction n'est cependant pas précisément quantifiable. Les FINN CHAMBERS® sont utilisés pour tester toutes les catégories d'allergènes, que ce soit les allergènes de contact ou les autres. En particulier, le mélange allergène/cellulose préparé extemporanément peut contenir par exemple des aliments pour la recherche d'une allergie alimentaire, du pollen pour la recherche d'une allergie respiratoire, un colorant ou un métal pour la recherche d'une allergie de contact.

Si cette méthode permet d'utiliser des allergènes d'une variété infinie, elle présente cependant l'inconvénient d'être difficile d'emploi. En effet, un certain nombre d'erreurs peuvent apparaître du fait par exemple :
- que le support de cellulose est déplacé au cours de la mise en place ;
- que le mélange allergéniques, en trop grande quantité, vient contaminer les alvéoles voisines ;
- que l'allergène est en trop faible concentration pour provoquer une réaction allergique.
- que la quantité d'allergène utilisée est variable et ne permet pas la standardisation du test.

Par ailleurs, si le test est utilisé pour détecter plusieurs allergènes, apparaît un risque de confusion au cours de l'interprétation, du fait de l'absence de repérage sur les supports adhésifs des allergènes utilisés. En outre et surtout, ce type de test nécessite de disposer d'allergènes frais ou en suspension, qui doivent être solubilisés ou dispersés dans un solvant extemporanément, c'est à dire avant application du test sur la peau.

En conclusion, l'ensemble des manipulations nécessaires rend ce test aléatoire et le réserve aux centres spécialisés disposant de personnel entraîné. Dès lors, l'utilisation de ce type de test en routine est particulièrement limitée, notamment dans les cabinets médicaux.

Un patch connu sous la dénomination LEUKOTEST® est réalisé selon un principe similaire au FINN CHAMBERS® précédemment décrites, à la différence près que les alvéoles en PVC sont incluses dans le support adhésif et non pas collées sur le support adhésif et contiennent de la cellulose non pas amovible, mais restant fixée à la coupelle.

De même que précédemment, la préparation du test est effectuée extemporanément avec des allergènes frais ou en suspension prête à l'emploi. Plus facile d'emploi que les FINN CHAMBERS®, elle comporte cependant de nombreux risques d'erreurs de manipulation. On note ainsi :
- l'absence de contrôle de la quantité d'allergène introduite dans chaque alvéole ;
- l'absence d'indication concernant la nature des allergènes utilisés sur les supports plastiques ;
- et également la nécessité de disposer des allergènes sous forme adéquate pour être déposés sur le support cellulosique.

Un second type de patch est connu sous la dénomination TRUE TEST® et décrit plus précisément dans le document US-A-4 836 217. Le TRUE TEST® supprime la présence des cupules métalliques, qu'il substitue par un gel, dans lequel sont incorporés les allergènes, le gel étant directement appliqué sur une bande adhésive. Les allergènes, dont les seuls concernés sont exclusivement les allergènes de contact, peuvent être incorporés dans le gel sous différentes formes. Ainsi, si l'allergène est soluble dans le solvant contenu dans le gel, alors l'allergène est directement incorporé dans le gel. En revanche, si l'allergène est insoluble, il est nécessaire de le disperser de la manière la plus homogène possible directement dans le gel. Le principal inconvénient de ce type de patch est qu'il nécessite un support cellulosique ou un gel, capable d'interagir avec l'allergène et qu'il n'assure aucune garantie de conservation des allergènes d'origine organique dans leur état réactogène d'origine.

Plus particulièrement appliqué au cas des allergènes, le premier problème que se propose de résoudre l'invention est de proposer un patch permettant de tester tous les allergènes, et d'autre part, de garantir la conservation des allergènes organiques dans leur état réactogène.

Un second problème que se propose de résoudre l'invention est de développer un patch prêt à l'emploi, c'est-à-dire un patch ne nécessitant aucune préparation extemporanée de l'allergène.

Un troisième problème que se propose de résoudre l'invention est de foumir un patch susceptible de contenir et de délivrer au contact de la peau une quantité déterminée d'allergène et constante d'un patch à l'autre, cette particularité assurant fiabilité et reproductibilité au test.

Pour ce faire l'invention propose un patch qui se caractérise en ce qu'il se présente sous forme d'un support doté de propriétés électrostatiques, dont la périphérie est enduite de matière adhésive faisant joint d'étanchéité, toute ou partie de la surface non adhésive du support étant directement recouverte d'au moins une substance biologiquement active sous forme de particules individualisées ou agglomérées, lesdites particules étant maintenues au contact de la surface non adhésive du support par des forces électrostatiques.

Dans un mode de réalisation préféré, la substance biologiquement active se présente sous forme d'un allergène.

Le patch de l'invention permet donc d'utiliser les allergènes, sous forme de particules à l'état pur ou après transformation, permettant ainsi de concerner tout les allergènes et ce, quel que soit la consistance et la forme de l'allergène à l'état frais. Par ailleurs, l'utilisation d'allergènes sous forme pure, native, entière ou fractionnée, c'est à dire dans leur état réactogène d'origine, et-en l'absence de tout ajout de gel, de solvant ou de support, permet non seulement de disposer d'un patch n'altérant pas laconservation de l'allergène, mais également d'un patch prêt à l'emploi, en dehors préparation de la peau préalablement à son application.

Par ailleurs et dans une forme de réalisation avantageuse, le support présente un dispositif de marquage de l'allergène permettant ainsi d'éviter à l'utilisateur toute erreur, lors de l'application sur la peau ou de l'ablation du patch. Le dispositif de marquage peut se présenter sous la forme d'un marquage imprimé au dos du support, d'un tatouage provisoire laissé à la surface de la peau au moment de l'ablation du patch ou encore d'une pastille autocollante maintenue sur la partie adhésive du support et désolidarisée de celui-ci au moment de l'ablation du patch.

S'agissant de la forme de l'allergène à l'état frais, trois hypothèses sont possibles. Dans une première hypothèse, l'allergène naturel se présente sous forme de poudre c'est à dire déjà sous forme de particules individualisées, de sorte qu'il n'est pas forcément nécessaire de le transformer (par exemple farine de blé) hormis, peut-être, en réduisant la taille des particules.

Dans une seconde hypothèse, l'allergène se présente sous forme solide de plus ou moins grande taille. Dans ce cas, il est nécessaire de le réduire en particules individualisées, éventuellement après transformation visant à assurer sa conservation sans dénaturation. C'est le cas par exemple de la cacahuète dans le cas d'une allergie alimentaire à l'arachide.

Dans une troisième hypothèse, l'allergène naturel se présente sous forme liquide. C'est le cas par exemple du lait, incriminé -également dans certaines allergies alimentaires, qui doit dans ce cas être lyophilisé pour obtenir une forme poudre. Dans certains cas, il sera nécessaire de n'employer que l'une des fractions purifiées de l'allergène à tester. C'est le cas par exemple de la fraction protéique de l'oeuf, l'ovalbumine ou du lait de vache, voire des meules protéines du lactosérum extraites du lait de vache

Dans le cas des allergènes liquides, la forme poudre peut être obtenue par les techniques connues telles que par exemple, lyophilisation (congélation et sublimation sous vide) ou chauffage et atomisation, le choix de ces techniques, notamment le degré de micronisation, étant laissé à l'appréciation de l'homme du métier en fonction des caractéristiques physico-chimiques de l'allergène considéré.

Pour assurer la conservation du patch dans son emballage et notamment éviter l'altération de l'allergène par l'air ambiant, les particules subissent un traitement particulier, tel que lyophilisation, pasteurisation, ionisation et plus généralement tout traitement connu de l'homme du métier. Quelle que soit la substance biologiquement active qu'il renferme, le patch est en outre confectionné et/ou conservé sous vide et présente, en regard du support, une étiquette pelable destinée à être retirée avant application du patch sur la peau.

Selon une caractéristique essentielle de l'invention, le patch de l'invention comprend un support doté de propriétés électrostatiques.

Dans la suite de la description et dans les revendications, par l'expression « support électrostatique », on désigne tout support fabriqué en un matériau susceptible d'accumuler des charges électrostatiques et de les conserver en développant ainsi des forces de maintien, notamment par frottement, chauffage, ionisation ou toute autre technique connue de l'homme du métier, les charges apparaissant d'un côté ou de l'autre de à la surface du support, peuvent être positives ou négatives en fonction du matériau constituant ledit support et du moyen utilisé pour les faire apparaître. Dans tous les cas, les charges positives ou négatives réparties sur la surface du support sont à l'origine de forces d'attraction sur des matériaux conducteurs ou non, dans le cas d'espèce sur l'allergène sous forme de particules individualisées ou agglomérées. Il peut aussi arriver que ce soit les dites particules qui sont ionisées, ce qui peut provoquer alors le même type de forces d'attraction électrostatique entre ces particules et le support. Aussi, l'un des avantages du patch de l'invention est de permettre un dosage précis de la masse surfacique de l'allergène ou plus généralement de la substance biologiquement active déposé et constant d'un lot à l'autre en fonction :
- d'une part, du choix du support et de sa capacité à stocker les charges électriques sur sa surface ;
- du type de particules d'allergène,
- et d'autre part, du flux de particules lors de la phase de dépose de l'allergène sur la surface non adhésive du support.

En pratique, tout matériau peut être utilisé comme support pourvu qu'il ait les qualités électrostatiques requises. En particulier, le support est constitué de verre ou d'un polymère choisi dans le groupe comprenant les plastiques cellulosiques (CA, CP), le polychlorures de vinyle (PVC), les polypropylènes, les polystyrènes, les polycarbonates, les polyacrylique, en particulier le polyméthacrylate de méthyle (PMMA), les polymères fluorés (PTFE par exemple), etc Cette liste n'est en aucun cas limitative. Le verre sera avantageusement choisi si l'on désire un support particulièrement hypoallergénique.

En conséquence, le support peut être rigide ou souple, hydrophile ou non, translucide ou non, en fonction de la matière constituante. Dans la cas du verre, le support peut alors être rendu incassable en collant sur le verre, une feuille de plastique.

Dans un mode de réalisation avantageux, on choisit un support transparent, permettant de le cas échéant de constater directement l'apparition d'une réaction, sans avoir à nécessairement retirer le patch.

Pour affiner d'avantage encore la détection de la réaction inflammatoire, le patch est muni, au niveau de la surface adhésive ou de la surface non adhésive, d'un dispositif sensible aux réactions physico-chimiques de la peau constatées au cours de la réaction inflammatoire locale induite par une réaction positive. Il peut s'agir d'un indicateur coloré sensible aux variations locales de pH par exemple. On peut dans ce cas envisager un système de lecture facilitant l'interprétation, indépendant de la réaction locale

Pour réactiver les forces électrostatiques à la surface du support et ainsi renforcer les liaisons de la substance biologiquement active, en particulier de l'allergène avec le support juste avant la pose, le dos du support est revêtu d'une étiquette pelable, à retirer lors de la pose, laquelle permet en outre de conserver les allergènes à l'abri de la lumière.

Le transport des molécules d'allergènes ainsi stockées sur le patch est assuré par la sueur sécrétée par la peau dans l'enceinte délimitée par le patch et qui vient se charger de molécules d'allergènes à leur contact. L'efficacité du patch et donc sa sensibilité est donc grandement conditionnée par la création d'une phase liquide, dans laquelle l'allergène est en solution ou en suspension, favorisant ainsi son passage à travers les pores. Dès lors, le patch doit présenter des caractéristiques permettant de résoudre les deux problèmes suivants :
- empêcher l'évaporation de la sueur,
- et permettre la circulation de la sueur.

S'agissant du problème de l'évaporation de la sueur, celle-ci-est obtenue d'une part, grâce à l'imperméabilité du support, et d'autre part, grâce à la zone adhésive périphérique faisant joint d'étanchéité et délimitant un espace hermétiquement fermé entre la peau et le support.

Pour résoudre le problème de la circulation de la sueur, le support présente avantageusement, dans la zone non enduite de matière adhésive, une dépression formant creux. Cette dépression est obtenue par formatage à chaud ou à froid et permet de déposer l'allergène dans le creux, de sorte que ledit allergène n'est pas en pression sur la peau et n'altère pas la circulation sanguine dans la zone considérée. Cette dépression permet en outre de ne pas mettre l'allergène au contact de la couche pelable retirée avant l'application du patch sur la peau. Avantageusement, l'enceinte définie pas le creux est maintenue sous vide.

En d'autres termes, l'allergène maintenu originellement et de manière réversible sur le support par des forces électrostatiques est libéré dans sa totalité dans la cavité ainsi obtenue et se mêle à la sueur, laquelle est plus facilement sécrétée du fait de l'augmentation de la chaleur locale et de l'hypervascularisation qui en découle. La pénétration de l'allergène par les pores de la peau est donc facilitée et l'hypervascularisation permet en outre l'afflux d'éléments immunologiquement compétents. La lecture se fait après ablation du support plastique et observation d'un délai suffisant de manière à éliminer l'érythème non spécifique provoqué par le matériau adhésif.

Pour permettre le déclenchement de la réaction cutanée, les particules sont réparties sur le support à raison d'une quantité qui dépend de l'allergène concerné. Comme déjà dit, l'avantage du patch est de proposer un dispositif contenant une quantité déterminée d'allergène, délivré en totalité ce qui permet de standardiser les patchs. Pour le lait par exemple, les particules sont réparties sur le support à raison d'une quantité comprise entre 0.001 et 1 g/cm².

L'invention concerne également un kit de patch comprenant une pluralité de patch tel que décrit précédemment, chaque patch du kit contenant une quantité croissante d'allergène permettant ainsi d'augmenter les doses d'allergènes au cours d'une cure de désensibilisation.

La mise en oeuvre des allergènes sous forme de particules fixées directement sur le support à l'état sec présente-en outre les avantages suivants.

Elle permet tout d'abord d'éviter toute interaction chimique ou toute réaction qui pourrait perturber le processus allergique ou fausser son diagnostic en ne mettant en contact avec la peau que les molécules incriminées. Par ailleurs, l'utilisation de l'allergène sous forme de particules permet de conserver l'allergène dans un emballage approprié, de sorte qu'il n'est plus utile de procéder à une préparation extemporanée. Enfin, le contact des particules avec la sueur exsudée par la peau permet d'obtenir une solution très concentrée favorisant une pénétration rapide des molécules à travers l'épiderme.

Bien entendu, le patch de l'invention est notamment apte à dépister l'état de sensibilisation à un allergène donné tout comme à plusieurs allergènes à la fois. Dans ce dernier cas, le support présente plusieurs zones douées de propriétés électrostatiques, avantageusement sous forme de creux, recouvertes chacune d'un allergène à tester différent, chaque zone électrostatique étant séparée par une zone non électrostatique

Selon un autre mode de réalisation, le patch présente dans une même zone, un mélange de plusieurs allergènes permettant de dépister l'état de sensibilisation d'un sujet à une série d'allergènes donnés. Ce peut être intéressant par exemple pour déterminer l'état de sensibilisation d'un sujet à une série d'allergènes alimentaires. Dans le cas de l'association de plusieurs allergènes, soit disposés sur des supports électrostatiques séparés, soit mélangés sur le même support, le choix des allergènes dépend des listes d'allergènes en cause dans les pathologies les plus fréquentes en accord avec les données de la littérature. Ce choix est effectué de manière à former des associations spécifiques à chaque cadre pathologique dans chacune des grandes tranches d'âge. Ces listes d'allergènes sont d'ailleurs susceptibles d'être modifiées en fonctions des habitudes alimentaires et des conditions d'environnement propres aux lieux de diffusion des patchs. Dans certains cas, les allergènes peuvent être choisis dans toute liste publiée par les autorités sanitaires.

L'invention concerne également l'utilisation du patch précédemment décrit pour le dépistage de l'état de sensibilisation d'un sujet à un allergène, consistant à appliquer le patch sur la peau, puis après retrait, à détecter la présence ou l'absence d'une réaction cutanée.

Dans une forme de réalisation avantageuse, le patch est utilisé pour le dépistage de l'état de sensibilisation d'un sujet à un allergène alimentaire contenu dans les produits choisis dans le groupe comprenant le lait de vache, l'oeuf, le blé, la cacahuète.

Dans une autre forme de réalisation, le patch est utilisé pour le dépistage d'un sujet sensible à l'allergène contenu dans le latex.

Le patch de l'invention peut par ailleurs être utilisé pour la désensibilisation d'un sujet à un ou plusieurs allergènes donnés. Dans ce cas, le patch est appliqué sur la peau pour une durée déterminée en fonction de la quantité d'allergène à délivrer. Des patchs contenant des quantités croissantes d'allergènes peuvent être utilisés. Une libération programmée de l'allergène à partir du patch peut être avantageusement envisagée.

Le patch de l'invention peut être utilisé lors du diagnostic de l'allergie de contact par la mise au contact de la peau de tout allergène de contact -sans adjonction de gel, buvard ou solvant.

Il peut également servir à l'administration de toute substance biologiquement active en vue d'obtenir une action thérapeutique (médicament) ou préventive (vaccin) ou cosmétique.

L'invention et les avantages qui en découlent ressortiront bien de l'exemple de réalisation qui suit à l'appui des figures annexées.
La figure 1 est une représentation schématique d'un mode de réalisation préféré de l'invention.
La figure 2 est une vue en coupe selon la figure 1.

### Exemple 1

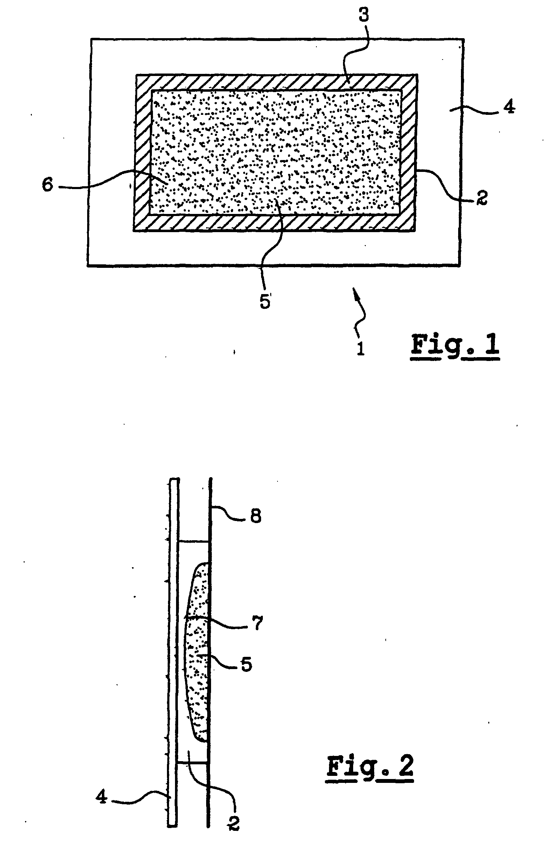

On a représenté sur la figure 1 le patch de l'invention défini par la référence générale (1). Selon une première caractéristique, ce patch est constitué d'un support réalisé en acétate de cellulose (2) dont la périphérie (3) est enduite d'une matière adhésive. Le dos du support (2) est en outre recouvert lui-même d'une étiquette pelable (4). Comme le montre cette figure, l'allergène (5) est réparti sous forme pulvérulente sur toute le zone (6) non adhésive du support en acétate (2).

Comme le montre la figure 2, le support en acétate (2) présente sur toute la surface non adhésive une dépression (7), dans laquelle sont réparties les particules individualiées. Le patch présente par ailleurs une seconde étiquette pelable (8) destinée à venir en regard de l'ensemble support acétate et pellicule isolante (4). Cette feuille pelable est bien entendu retirée avant application du patch sur la zone considérée.

### Exemple 2

Dans cet exemple, on compare l'efficacité des patchs de l'invention avec des patchs de l'art antérieur du type FINN CHAMBERS®

### 1 - Moyens et Méthodes

Un patch de l'invention est appliqué sur le dos de 15 enfants. Ces enfants présentent des signes faisant suspecter une allergie aux protéines de lait de vache (APLV).

Le patch présente deux zones. Une première zone supérieure constituée d'un support adhésif sur lequel est déposé un comprimé composé de poudre de lait écrémé sans aucun autre élément associé, ce qui correspond au patch de l'invention. La zone inférieure est constituée du support adhésif sur lequel est déposée une cupule, dont le fond est rempli de lait écrémé dilué absorbé sur un support cellulosique du type FINN CHAMBERS®.

La lecture est effectuée 48 heures plus tard, après ablation de l'adhésif. La présence de réaction érythémateuse ou maculaire signe la positivité.

### 2 - Résultats

15 enfants âgés de 5 semaines à 11 ans et 3/12 ont bénéficié d'une recherche d'allergie au lait de vache à l'aide d'un patch double. La réaction obtenue a été évaluée 48 heures après la pose du patch.

Tous les enfants présentaient des signes cliniques suggérant une possible allergie aux protéines du lait de vache RGO résistant aux moyens thérapeutiques habituels (9 cas), eczéma (6 cas), vomissements (2 cas), douleurs abdominales chroniques (2 cas), diarrhée chronique (3 cas), manifestations douloureuses inexpliquées (4 cas), malaises (1 cas).

Les deux tests étaient positifs dans 3 cas, négatifs dans 10 cas, le FINN CHAMBERS était positif et le patch négatif dans un cas-et, à l'inverse, le FINN CHAMBERS négatif et patch positif dans un cas.

### 3. Conclusion

Chez ces 15 enfants suspects d'allergie alimentaire aux protéines du lait de vache, le patch de la présente invention s'est révélé aussi sensible que le procédé FINN CHAMBERS. Dans deux cas les résultats se sont avérés discordants sans qu'il soit possible de distinguer les deux méthodes.

## Revendications

1. Patch (1) **caractérisé en ce qu'**il se présente sous forme d'un support (2) doté de propriétés électrostatiques, dont la périphérie (3) est enduite de matière adhésive faisant joint d'étanchéité, toute ou partie de la surface non adhésive du support étant directement recouverte d'au moins une substance (5) biologiquement active sous forme de particules individualisées ou agglomérées, lesdites particules étant maintenues au contact de la surface non adhésive du support par des forces électrostatiques.

2. Patch selon la revendication 1, **caractérisé en ce qu'**il est muni, en regard du support, d'une étiquette pelable (4) destinée à être retirée avant application sur la peau.

3. Patch selon l'une des revendications précédentes, **caractérisé en ce que** le support est constitué de verre ou d'un polymère choisi dans le groupe comprenant les plastiques cellulosiques (CA, CP), le polychlorures de vinyle (PVC), les polypropylènes, les polystyrènes, les polycarbonates, les polyacryliques.

4. Patch selon l'une des revendications précédentes, **caractérisé en ce que** le dos du support est revêtu d'une étiquette pelable.

5. Patch selon l'une des revendications précédentes, **caractérisé en ce que** le support présente, dans la zone non enduite de matière adhésive, une dépression (7) formant creux.

6. Patch selon l'une des revendications précédentes, **caractérisé en ce que** la substance active se présente sous forme d'un allergène.

7. Patch selon la revendication 6, **caractérisé en ce que** l'allergène a subi un traitement de lyophilisation, pasteurisation ou ionisation.

8. Patch selon l'une des revendications 6 ou 7, **caractérisé en ce que** l'allergène se présente sous forme d'une fraction allergénique purifiée.

9. Patch selon l'une des revendications 6 à 8, **caractérisé en ce que** le support est muni d'un dispositif de marquage de l'allergène se présentant sous la forme d'un marquage imprimé au dos du support, d'un tatouage provisoire laissé à la surface de la peau au moment de l'ablation du patch ou encore d'une pastille autocollante maintenue sur la partie adhésive du support et désolidarisée de celui-ci au moment de l'ablation du patch.

10. Patch selon l'une des revendications 6 à 9, **caractérisé en ce qu'**il est muni, au niveau de la zone adhésive ou de la surface non adhésive, d'un dispositif sensible aux réactions physico-chimiques de la peau constatées au cours de la réaction inflammatoire locale induite par une réaction positive.

11. Patch selon la revendication 10, **caractérisé en ce que** ledit dispositif se présente sous forme d'un indicateur coloré sensible aux variations locales de pH.

12. Patch selon l'une des revendications 6 à 11, **caractérisé en ce que** le support présente plusieurs zones douées de propriétés électrostatiques recouvertes chacune d'un allergène différent, chaque zone électrostatique étant séparée par une zone non électrostatique.

13. Kit de patch comprenant une pluralité de patch selon l'une des revendications 1 à 12, caractérisé en ce chaque patch contient une quantité croissante d'allergènes.

## Claims

1. A patch (4), **characterized in that** it is in the form of a support (2) having electrostatic properties, the periphery (3) of which is coated with an adhesive material which makes an airtight joint, all or part of the non-adhesive surface of the support being directly covered with at least one biologically active substance (5) in the form of individualized or agglomerated particles, said particles being kept in contact with the non-adhesive surface of the support as a result of electrostatic forces.

2. The patch as claimed in claim 1, **characterized in that** it has, opposite the support, a label (4) which can be peeled off, intended to be removed before application to the skin.

3. The patch as claimed in either of the preceding claims, **characterized in that** the support consists of glass or of a polymer chosen from the group comprising cellulose plastics (CA, CP), polyvinyl chlorides (PVCs), polypropylenes, polystyrenes, polycarbonates and polyacrylics.

4. The patch as claimed in one of the preceding claims, **characterized in that** the back of the support is covered with a label which can be peeled off.

5. The patch as claimed in one of the preceding claims, **characterized in that** the support has, in the area not coated with adhesive material, a depression (7) forming a hollow.

6. The patch as claimed in one of the preceding claims, **characterized in that** the active substance is in the form of an allergen.

7. The patch as claimed in claim 6, **characterized in that** the allergen has undergone a treatment of lyophilization, pasteurization or ionization.

8. The patch as claimed in either of claims 6 and 7, **characterized in that** the allergen is in the form of a purified allergenic fraction.

9. The patch as claimed in one of claims 6 to 8, **characterized in that** the support has an allergen marking device which is in the form of marking printed on the back of the support, of a temporary tattoo left at the surface of the skin when the patch is removed, or else of a self-adhesive disk maintained on the adhesive part of the support and separated therefrom when the patch is removed.

10. The patch as claimed in one of claims 6 to 9, **characterized in that** it has, in the adhesive area or in the non-adhesive area, a device sensitive to the physicochemical reactions of the skin noted in the course of the local inflammatory reaction induced by a positive reaction.

11. The patch as claimed in claim 10, **characterized in that** said device is in the form of a colored indicator sensitive to local variations in pH.

12. The patch as claimed in one of claims 6 to 11, **characterized in that** the support has several areas having electrostatic properties each covered with a different allergen, each electrostatic area being separated by a non-electrostatic area.

13. A patch kit comprising a plurality of patches as claimed in one of claims 1 to 12, **characterized in that** each patch contains an increasing amount of allergens.

## Patentansprüche

1. Pflaster (1), **dadurch gekennzeichnet, daß** es die Form eine Trägers (2) mit elektrostatischen Eigenschaften aufweist, dessen Umfang (3) mit einem Haftmaterial beschichtet ist, welches als Dichtung dient, wobei die nicht haftende Oberfläche des Trägers vollständig oder teilweise direkt mit mindestens einem biologischen Wirkstoff (5) in Form von vereinzelten oder agglomerierten Partikeln bedeckt ist, wobei die genannten Partikel mittels elektrostatischer Kräfte in Kontakt mit der nicht haftenden Oberfläche des Trägers gehalten werden.

2. Pflaster nach Anspruch 1, **dadurch gekennzeichnet, daß** es gegenüber dem Träger mit einem abziehbaren Aufkleber (4) versehen ist, welcher dazu bestimmt ist, vor dem Aufbringen auf die Haut entfernt zu werden.

3. Pflaster nach einem der vorangehenden Ansprüche, **dadurch gekennzeichnet, daß** der Träger aus Glas besteht oder aus einem Polymer, ausgewählt aus der Gruppe umfassend die Zellulosekunststoffe (CA, CP), Polyvinylchloride (PVC), Polypropylene, Polystyrole, Polycarbonate, Polyacryle.

4. Pflaster nach einem der vorangehenden Ansprüche, **dadurch gekennzeichnet, daß** die Rückseite des Trägers mit einem abziehbaren Aufkleber bedeckt ist.

5. Pflaster nach einem der vorangehenden Ansprüche, **dadurch gekennzeichnet, daß** der Träger in der nicht mit Haftmaterial beschichteten Zone eine Vertiefung (7) aufweist, welche einen Hohlraum bildet.

6. Pflaster nach einem der vorangehenden Ansprüche, **dadurch gekennzeichnet, daß** der Wirkstoff die Form eines Allergens aufweist.

7. Pflaster nach Anspruch 6, **dadurch gekennzeichnet, daß** das Allergen einer Gefriertrocknungs-, Pasteurisierungs- oder Ionisierungsbehandlung unterzogen wurde.

8. Pflaster nach einem der Ansprüche 6 oder 7, **dadurch gekennzeichnet, daß** das Allergen die Form einer gereinigten allergenen Fraktion aufweist.

9. Pflaster nach einem der Ansprüche 6 bis 8, **dadurch gekennzeichnet, daß** der Träger mit einer Vorrichtung zur Markierung des Allergens versehen ist, welche die Form einer auf die Rückseite des Trägers aufgedruckten Markierung aufweist, die Form einer provisorischen Tätowierung, welche zum Zeitpunkt des Ablösens des Pflasters auf der Hautoberfläche belassen wird, oder auch die eines selbstklebenden Plättchens, welches auf dem haftenden Teil des Trägers gehalten wird und von diesem im Zeitpunkt des Ablösens des Pflasters getrennt wird.

10. Pflaster nach einem der Ansprüche 6 bis 9, **dadurch gekennzeichnet, daß** es im Bereich der haftenden Zone oder der nicht haftenden Oberfläche mit einer Vorrichtung versehen ist, welche empfindlich ist für die physikalisch-chemischen Reaktionen der Haut, welche im Verlauf der durch eine positive Reaktion hervorgerufenen lokalen Entzündungsreaktion festgestellt werden.

11. Pflaster nach Anspruch 10, **dadurch gekennzeichnet, daß** die genannte Vorrichtung die Form eines für lokale Veränderungen des pH-Wertes empfindlichen gefärbten Indikators aufweist.

12. Pflaster nach einem der Ansprüche 6 bis 11, **dadurch gekennzeichnet, daß** der Träger mehrere mit elektrostatischen Eigenschaften versehene, jeweils mit einem unterschiedlichen Allergen bedeckte Zonen aufweist, wobei jede elektrostatische Zone durch eine nicht elektrostatische Zone abgetrennt ist.

13. Pflasterkit, welcher eine Mehrzahl von Pflastern nach einem der Ansprüche 1 bis 12 umfaßt, **dadurch gekennzeichnet, daß** jedes Pflaster eine gesteigerte Menge Allergene enthält.
